# EUROPEAN PATENT APPLICATION

(11) **EP 4 393 319 A1**
(43) Date of publication of application: **03.07.2024**
(21) Application number: 22861470.7
(22) Date of filing: 26.08.2022
(51) Int. Cl.: A23L 5/00, A23C 11/02, A23L 2/00, A23L 2/38, A23L 11/65

(54) **MODIFIER FOR FERMENTED VEGETABLE DRINKS AND FOODS**

(30) Priority: 27.08.2021 JP 2021138778
(71) Applicant: Amano Enzyme Inc., Nagoya-shi Aichi 460-8630 (JP)
(72) Inventor: KAMON, Masahiro, Kakamigahara-shi, Gifu 509-0109 (JP); TADA, Shusaku, Kakamigahara-shi, Gifu 509-0109 (JP); YAMASHIRO, Kan, Kakamigahara-shi, Gifu 509-0109 (JP); HAYASHI, Takuma, Kakamigahara-shi, Gifu 509-0109 (JP); HASHIMURA, Dai, Kakamigahara-shi, Gifu 509-0109 (JP)
(74) Representative: Forrest, Stuart
(86) International application number: PCT/JP2022/032267
(87) International publication number: WO 2023/027182

(57) **Abstract**

[Summary]

[Problems] To provide a technique for promoting fermentation during the production of a fermented plant-base drink or food and improving the stringiness of the produced fermented plant-base drink or food.

[Solution] The present technique provides a modifier for fermented plant-base drinks or foods, the modifier containing hemicellulase. The present technique also provides a method for producing a fermented plant-base drink or food, a method for promoting fermentation, and a method for improving stringiness, the methods comprising: an enzymatic action step for allowing hemicellulase to act on a portion or all of a plant-base raw material; and a fermentation step for performing fermentation.

## Description

### Technical field

The present invention relates to a modifier for fermented plant-base drinks or foods. More specifically, the present invention relates to a technique for promoting fermentation during the production of a fermented plant-base drink or food, and a technique for improving the stringiness of the produced fermented plant-base drink or food.

### Background Art

In recent years, plant-base proteins have been attracting attention, and there is a need for modification of taste and texture using enzymes. Furthermore, due to the health-conscious boom in recent years, interest in a fermented drink or food has increased significantly. Under these circumstances, the development of a manufacturing technique for fermented drinks and foods using plant-base proteins is also progressing.

For example, Patent Document 1 discloses a technique for producing lactic acid fermented soybean foods having good texture and flavor in which the fermentation time is shortened by adding a plant cell wall degrading enzyme during the production of lactic acid fermented soybean foods made from at least one of soybean flour, defatted soybeans, and soybean protein.

Even if a plant-base milk made from soybeans or the like is fermented using lactic acid bacteria, the viscosity and stringiness of milk-derived yogurt may not be obtained in some cases. On the other hand, for example, Patent Document 2 discloses a technique for producing a fermented soy milk product with high viscosity and stringiness by subjecting soy milk to neutral protease treatment and then fermenting it using lactic acid bacteria.

### Citation List

### Patent Literature

Patent Literature 1: Japanese Unexamined Patent Application Publication No. 2011-135832
Patent Literature 2: International Publication No. 2016/152590 pamphlet

### Summary of Invention

### Technical Problem

As mentioned above, various techniques are being developed to improve the fermentation efficiency and stringiness of a fermented drink or food using plant-base proteins, but the reality is that further development is desired.

Therefore, the main purpose of the present technique is to provide a technique that promotes fermentation during the production of a fermented plant-base drink or food and improves the stringiness of the produced fermented plant-base drink or food.

### Solution to Problem

The inventors of the present application have conducted extensive research into techniques for promoting fermentation during the production of a fermented plant-base drink or food and improving the stringiness of the produced fermented plant-base drink or food, and resultantly found that hemicellulase, an enzyme that had not received particular attention until now, has a stronger effect of promoting fermentation and a stronger effect of improving stringiness than other enzymes, leading to the completion of the present invention.

That is, the present technique first provides a modifier for fermented plant-base drinks or foods comprising hemicellulase.

The modifier for fermented plant-base drinks or foods according to the present technique has an effect of promoting fermentation.

Moreover, the modifier for fermented plant-base drinks or foods according to the present technique has an effect of improving stringiness.

As the hemicellulase used in the modifier for fermented plant-base drinks or foods according to the present technique, hemicellulase derived from a microorganism can be used.

The modifier for fermented plant-base drinks or foods according to the present technique can be used in fermented plant-base milk or plant-base lactic acid bacteria drinks.

In the present technique, next, provided is a method for producing a fermented plant-base drink or food, comprising:
an enzymatic action step for allowing hemicellulase to act on a portion or all of a plant-base raw material; and
a fermentation step for performing fermentation.

In the method for producing a fermented plant-base drink or food according to the present technique, the enzymatic action step can be performed before and/or simultaneously with the fermentation step.

In the fermentation step of the method for producing a fermented plant-base drink or food according to the present technique, lactic acid fermentation can be performed.

The present technique further provides a method for promoting fermentation of a fermented plant-base drink or food, and a method for improving stringiness of a fermented plant-base drink or food, the methods comprising:
an enzymatic action step for allowing hemicellulase to act on a portion or all of a plant-base raw material; and
a fermentation step for performing fermentation.

### Advantageous Effects of Invention

According to the present technique, it is possible to promote fermentation during the production of a fermented plant-base drink or food and to improve the stringiness of the produced fermented plant-base drink or food.

### Description of Embodiments

Hereinafter, preferred embodiments for carrying out the present invention will be described. Note that the embodiment described below is an example of a typical embodiment of the present invention, and the scope of the present invention should not be interpreted narrowly thereby.

### <1. Modifier for fermented plant-base drinks or foods>

The modifier for fermented plant-base drinks or foods according to the present technique contains hemicellulase as an active ingredient. Hemicellulase is an enzyme that hydrolyzes hemicellulose. Hemicellulose is a heteropolysaccharide, composed of multiple constituent sugars, and generally has a branched structure in which side chains are formed by other constituent sugars for the main chain from which the name derives. Specific examples of hemicellulose include mannan, β-1,3-1,4-glucan, glucomannan, xylan, xyloglucan, glucuronoxylan, and the like. Hemicellulase is an enzyme that degrades these hemicelluloses.

Specific examples of hemicellulase include xylanase, galactanase, mannanase, galactomannanase, arabinase, β-glucanase, etc., and these may be used in one type alone or in combination of two or more types.

Hemicellulase is not particularly limited in its origin, and may be derived from microorganisms such as basidiomycetes (genus Corticium, genus Pycnoporus), filamentous fungi (genus Aspergillus, genus Humicola, genus Penicillium, genus Trichoderma), actinomycetes (genus Streptomyces), bacteria (genus Bacillus), or the like, or may be an artificially synthesized enzyme. Moreover, these may be used in one type alone or in combination of two or more types. Among these, in the present technique, those derived from filamentous fungi are preferred, those derived from microorganisms of the genus Aspergillus are more preferred, and those derived from Aspergillus niger are even more preferred.

The content of hemicellulase in the modifier for fermented plant-base drinks or foods according to the present technique can be freely set as long as it does not impair the effects of the present technique. For example, it is preferable to contain hemicellulase in an amount of 0.5 U/mL or more, more preferably 1 U/mL or more, 2 U/mL or more, 4 U/mL or more, further preferably 6 U/mL or more, 8 U/mL or more based on the raw material of a fermented plant-base drink or food, and further, it is also possible to contain hemicellulase in an amount of 20 U/mL or more, 40 U/mL or more, or 80 U/mL or more.

Fermentation can be promoted by using the modifier for fermented plant-base drinks or foods according to the present technique during the production of a fermented plant-base drink or food. Furthermore, by using the modifier for fermented plant-base drinks or foods according to the present technique at the time of manufacturing a fermented plant-base drink or food, the stringiness of the manufactured fermented plant-base drink or food can be improved. In particular, when fermenting plant-base raw materials, there has been a problem that the viscosity and stringiness may be inferior compared to fermented drinks and foods produced by using animal raw materials such as milk, but by using the modifier for fermented plant-base drinks or foods according to the present technique during the production of fermented plant-base milk or plant-base lactic acid bacteria drinks, the stringiness of the produced fermented plant-base milk and plant-base lactic acid bacteria drinks can be improved. That is, the modifier for fermented plant-base drinks or foods according to the present technique can be suitably used for fermented plant-base milk or plant-base lactic acid bacteria drinks.

The modifier for fermented plant-base drinks or foods according to the present technique may be composed only of hemicellulase, or one or more types of other components may be freely selected to be contained as long as the effects of the present technique are not impaired. As other components, for example, components such as excipients, pH adjusters, coloring agents, flavoring agents, disintegrants, lubricants, stabilizers, and the like that are commonly used in formulation can be used. Furthermore, components having functions that are known or that will be discovered in the future may be used in combination depending on the purpose.

### <2. Method for producing a fermented plant-base drink or food>

The method for producing a fermented plant-base drink or food according to the present technique is a method of performing at least an enzymatic action step and a fermentation step. In addition, it is also possible to perform steps necessary for the production of general plant-base food or drink products, such as a recovery step for fermented plant-base food or drink products, etc., to the extent that the effects of the present technique are not impaired, before, after, or simultaneously with each step, depending on the type of fermented plant-base drinks or foods, etc. Each step will be explained in detail below.

### (1) Enzyme action step

The enzymatic action step is a step for allowing hemicellulase to act on a portion or all of the raw material of the fermented plant-base drink or food. In the present technique, by allowing hemicellulase to act on the raw material, the fermentation time in the fermentation step described below can be shortened. Moreover, the stringiness of the produced fermented plant-base drink or food can be improved.

Various conditions for the enzyme action step can be freely set as long as they do not impair the effects of the present technique. For example, the pH, temperature, action time, etc. can be set depending on the physicochemical properties of the hemicellulase used, such as the optimum pH, stable pH range, optimum temperature, temperature stability, and the like. The pH can be set, for example, to pH 3.0 to 10.0, preferably pH 5.0 to 7.5, more preferably pH 6.0 to 7.0. The temperature can be set, for example, to 20°C to 60°C, preferably 25°C to 55°C, more preferably 30°C to 45°C, even more preferably 30°C to 40°C. The action time can be set, for example, from 1 to 30 hours, preferably from 1.5 to 20 hours, and more preferably from 4 to 10 hours.

The amount of hemicellulase added in the enzymatic action step can be freely set as long as it does not impair the effects of the present technique. It is preferable to add hemicellulase in an amount of 0.5 U/mL or more, more preferably 1 U/mL or more, 2 U/mL or more, 4 U/mL or more, further preferably 6 U/mL or more, 8 U/mL or more based on the raw material of a fermented plant-base drink or food, and further, it is also possible to add hemicellulase in an amount of 20 U/mL or more, 40 U/mL or more, or 80 U/mL or more.

In the enzymatic action step, other enzymes can be used in addition to hemicellulase as long as the effects of the present technique are not impaired. For example, one or more enzymes selected from lactase, protease, transglutaminase, peroxidase, catalase, lipase, cellulase, glucose oxidase, carbohydrate oxidase, and amylase can be used in combination.

### (2) Fermentation step

The fermentation step is a step of fermenting a portion or all of the raw material for the fermented plant-base drink or food. The fermentation step can be performed after the enzyme action step or simultaneously with the enzyme action step.

In the fermentation step of the present technique, fermentation can be performed using one or more types of microorganisms selected from anaerobic microorganisms, aerobic microorganisms, and facultative anaerobic microorganisms. As the microorganisms that can be used in the present technique, one or more types of microorganisms that can be used in the production of general fermented drinks or foods can be freely selected and used, depending on the type of the fermented plant-base drink or food to be produced, as long as the effects of the present technique are not impaired. For example, the microorganisms include lactic acid bacteria, yeast, filamentous fungi, Bacillus subtilis, Acetobacteraceae, and the like.

In the fermentation step of the present technique, lactic acid fermentation can be performed. As the lactic acid bacteria used during lactic acid fermentation, one or more types of lactic acid bacteria that can be used in the production of general lactic acid fermented drinks or foods can be freely selected and used, depending on the type and purpose of the lactic acid fermented drink or food to be produced, as long as the effects of the present technique are not impaired. Examples of lactic acid bacteria include lactic acid cocci belonging to Lactococcus, Streptococcus, Pediococcus, and Leuconostoc; lactic acid bacillus belonging to Lactobacillus; Bifidobacterium, etc., and preferably, Lactococcus delbrueckii subsp. delbureckii.

Various conditions for performing the fermentation step can be freely set as long as they do not impair the effects of the present technique. For example, fermentation conditions, heating conditions, etc. can be set depending on the type of microorganism used. The fermentation temperature can be set to, for example, 20°C to 50°C, preferably 25°C to 45°C, more preferably 30°C to 40°C. The fermentation time can be set, for example, from 1 to 30 hours, preferably from 2 to 20 hours, and more preferably from 4 to 10 hours. The heating conditions include, for example, high temperature short time sterilization (HTST) method, ultra high temperature cooking (UHT) method, retort method, etc., and any condition may be used as long as the temperature of the food/drink material is 90°C or higher, preferably about 95°C. Examples thereof include a method in which food or drink materials are treated at 90°C to 100°C for 1 to 5 minutes, and a method in which food or drink materials are treated at 90°C to 95°C for 1 to 3 minutes.

In the present technique, by performing the enzyme action step, the time for the fermentation step can be shortened. Specifically, for example, the fermentation time can be shortened by one hour or more compared to the case where the enzyme action step is not performed. In other words, with this technique, the fermentation step can be performed in a shorter time than the case where the enzyme action step is not performed, and specifically, for example, the fermentation step can be performed in a shorter time by one hour or more than the case where the enzymatic action step is not performed.

### (3) Recovery step

The recovery step is a step of recovering the fermented plant-base drink or food that have undergone the enzyme action step and the fermentation step. As a specific recovery method, one type or a combination of two or more types of recovery methods used in the production of general plant-base drinks or foods can be used, depending on the type of the fermented plant-base drink or food to be produced.

The fermented plant-base drink or food that has undergone the enzyme action step and the fermentation step is characterized by high stringiness. Specifically, the fermented plant-base drink or food that has undergone the enzyme action step and the fermentation step has an outflow time of more than 30 seconds in the Zahn cup method. That is, with the present technique, it is possible to perform a recovery step to recover plant-base fermented drinks or foods with improved stringiness, and specifically, a recovery step can be performed to recover a fermented plant-base drink or food whose outflow time in the Zahn cup method is more than 30 seconds.

Further, in the present technique, by performing the enzyme action step, the stringiness of the fermented plant-base drink or food can be improved. Specifically, for example, the outflow time in the Zahn cup method can be extended by more than 30 seconds compared to the case where the enzyme action step is not performed. In other words, with the present technique, it is possible to carry out a recovery step of recovering a plant-base fermented drink or food with improved stringiness compared to the case where the enzyme action step is not performed, and specifically, for example, it is possible to carry out a recovery step of recovering a fermented plant-base drink or food with the outflow time in the Zahn cup method extended by more than 30 seconds compared to the case where the enzymatic action step is not performed.

By using the method for producing a fermented plant-base drink or food explained above, various fermented plant-base drinks or foods can be efficiently produced. In the present technique, "fermented plant-base drink or food" refers to a drink or food obtained by fermenting plant-base drink or food materials. Specific examples thereof include fermented plant-base milk (plant-base yogurt), plant-base lactic acid bacteria drinks, plant-base yogurt paste, plant-base cheese-like foods, alcoholic drinks (sake, beer, wine, shochu, etc.), various fermented seasonings (soy sauce, miso, vinegar, etc.), rice bran pickles, kimchi, natto, kuzu mochi, etc. Furthermore, in the present technique, "fermented plant-base drink or food" also includes secondary processed drinks or foods produced by using these fermented plant-base drinks or foods.

In the present technique, "fermented plant-base milk" refers to one obtained by fermentation of plant-base milk, and the plant-base milk includes plant-base milk products derived from, for example, peas, soybeans, fava beans, chickpeas, barley, wheat, oats, rice, buckwheat, Japanese millet, foxtail millet, hemp, algae, almonds, cashews, hazelnuts, pecans, macadamia nuts, pistachios, walnuts, Brazil nuts, peanuts, and coconuts.

In each step of the method for producing a fermented plant-base drink or food according to the present technique, raw materials used in the production of general food or drink products can be freely selected and used, as long as the effects of the present technique are not impaired, in addition to plant-base food or drink materials (plant-base milk, grains, vegetables, fruits, beans, etc.), various enzymes, and various microorganisms. For example, various seasonings, pH adjusters, coloring agents, flavoring agents, stabilizers, etc. can be used. Furthermore, when producing a secondary processed drink or food using a fermented plant-base drink or food, it is possible to use not only plant-base materials but also animal-based materials such as animal milk, seafood, meat, and the like.

### <3. Method for promoting fermentation of fermented plant-base drink or food and method for improving stringiness>

The method for promoting fermentation and the method for improving stringiness of a fermented plant-base drink or food according to the present technique are methods in which at least an enzyme action step and a fermentation step are performed. In addition, it is also possible to carry out steps that are used in the production and modification methods of general plant-base drinks or foods, such as, for example, a recovery step of a fermented plant-base drink or food, and the like, to the extent that the effects of the present technique are not impaired, before, after, or simultaneously with each step, depending on the type of the fermented plant-base drink or food, etc. Note that details of the enzyme action step, the fermentation step, and the recovery step carried out in the method for promoting fermentation and the method for improving stringiness of a fermented plant-base drink or food according to the present technique are the same as the enzyme action step, the fermentation step, and the recovery step in the method for producing a fermented plant-base drink or food described above, and therefore, their explanation will be omitted here.

Note that the present technique can also take the following configuration.
(1) A modifier for fermented plant-base drinks or foods, the modifier comprising hemicellulase.
(2) The modifier for fermented plant-base drinks or foods according to (1), which has an effect of promoting fermentation.
(3) The modifier for fermented plant-base drinks or foods according to (1) or (2), which has an effect of improving stringiness.
(4) The modifier for fermented plant-base drinks or foods according to any one of (1) to (3), wherein the hemicellulase is a hemicellulase derived from a microorganism.
(5) The modifier for fermented plant-base drinks or foods according to any one of (1) to (4), wherein the fermented plant-base drink or food is a fermented plant-base milk or a plant-base lactic acid bacteria drink.
(6) A method for producing a fermented plant-base drink or food, the method comprising:
   an enzymatic action step for allowing hemicellulase to act on a portion or all of a plant-base raw material; and
   a fermentation step for performing fermentation.
(7) The method for producing a fermented plant-base drink or food according to (6), wherein the enzyme action step is performed before and/or simultaneously with the fermentation step.
(8) The method for producing a fermented plant-base drink or food according to (6) or (7), wherein lactic acid fermentation is performed in the fermentation step.
(9) A method for promoting fermentation of a fermented plant-base drink or food, the method comprising:
   an enzymatic action step for allowing hemicellulase to act on a portion or all of a plant-base raw material; and
   a fermentation step for performing fermentation.
(10) A method for improving the stringiness of a fermented plant-base drink or food, the method comprising:
   an enzymatic action step for allowing hemicellulase to act on a portion or all of a plant-base raw material; and
   a fermentation step for performing fermentation.

### (EXAMPLES)

Hereinafter, the present invention will be explained in more detail based on Examples. It should be noted that the embodiment described below shows one example of a typical embodiment of the present invention, and the scope of the present invention should not be interpreted narrowly thereby.

In addition, in this technique, the following measurement method was used as a hemicellulase activity measurement method.

### [Activity measurement method]

An appropriate amount of enzyme was weighed, dissolved or uniformly dispersed with water, and diluted appropriately to obtain a sample solution. Zero point five zero grams (0.50 g) of xylan was weighed out, about 30 mL of water was added, and the solution was heated while stirring, and boiled for 3 minutes after it started to boil. After cooling, water was added to this solution to make 50 mL, which was used as a substrate solution. One milliliter (1 mL) of the substrate solution was weighed into a test tube, 3 mL of a 0.1 mol/L acetate buffer (pH 4.5) was added, and the mixture was warmed at 40°C for 10 minutes, then, 1 mL of a sample solution was added and shaken, and the solution was heated at 40°C for 30 minutes. Two milliliters (2 mL) of Somogyi test solution (III) was added to this solution and mixed, the test tube was stoppered, heated in a boiling water bath for 20 minutes, and immediately cooled. After cooling, 1 mL of Nelson's test solution was added to this solution, and the mixture was shaken well until the red precipitate was completely dissolved, and after being left at room temperature for about 20 minutes, water was added to make up to 25 mL. This solution was centrifuged at 25°C and 3,000 revolutions per minute for 10 minutes, and the supernatant was used as a test solution.

Separately, 1 mL of the substrate solution was weighed into a test tube, 3 mL of a 0.1 mol/L acetate buffer (pH 4.5) and 2 mL of Somogyi test solution (III) were added and shaken, then, 1 mL of a sample solution was added, and the test tube was stoppered, and the mixture was heated in a boiling water bath for 20 minutes and immediately cooled. The following procedure was performed in the same manner as in the preparation of the test solution, to obtain a comparison solution.

The absorbance at a wavelength of 500 nm was measured for the test solution and the comparison solution using water as a control. Under these conditions, the enzyme activity was calculated by setting the amount of enzyme producing reducing sugar equivalent to 1 mg of xylose per minute as 100 units.

### <Experimental Example 1>

In Experimental Example 1, the effect on fermentation time when hemicellulase was used before fermentation of plant-base raw materials was investigated. Soybeans (soy milk) were used as the plant-base raw material.

### (1) Experimental method

Four hundred grams (400 g) of ordinary water was added to 100 g of soybeans, and the soybeans were soaked for 24 hours. The soaked soybeans were taken out and 100 g was pulverized using a mixer (about 2 minutes) to prepare a pulverized soybean product (gojiru soup). While adding 7 times as much water as the pulverized soybean (gojiru soup) to the obtained pulverized soybean (gojiru soup), the resulting soybean was crushed further using a mixer to prepare a crushed soybean liquid. The resulting crushed soybean liquid was placed in a pot, brought to a boil, and then heated over medium to low heat for 10 minutes while stirring with a spatula to prevent burning. After cooling, hemicellulase derived from Aspergillus niger ("Hemicellulase 'Amano' 90" manufactured by Amano Enzyme Inc.) was added at a concentration of 0.1 (w/v)% (90 U/mL) and this was stirred at 50°C for 2 hours to perform enzyme treatment. After deactivation treatment was performed at 95°C for 5 minutes, the mixture was cooled, placed in a strainer bag, and the enzyme-treated crushed soybean liquid was strained to prepare enzyme-treated soybean milk. Thirty milliliters (30 mL) of the prepared enzyme-treated soy milk was dispensed into a 50 mL tube and sterilized at 95°C for 5 minutes.

The sterilized enzyme-treated soy milk was cooled with water, and then 0.6 mL of lactic acid bacteria starter containing Lactococcus delbrueckii subsp. delbureckii was added and fermentation was carried out at 37°C overnight in a clean bench. During fermentation, pH was monitored with a pH sensor. Note that the example in which hemicellulase treatment was not performed was designated as an enzyme-free group.

### (2) Results

The results are shown in Table 1.

**[Table 1]**

| Amount of hemicellulase (U/mL) | Time to reach pH 4.5 | Shortened time |
|---|---|---|
| Enzyme-free | 9.3 hrs | - |
| 90 U/mL | 7.3 hrs | 2 hrs |

### (3) Consideration

As shown in Table 1, it took 9.3 hours to reach pH 4.5, which is the point at which curd of the soy milk raw material is formed, in the enzyme-free group, but it was observed that it took 7.3 hours for the hemicellulase-added group, which means that fermentation time was shortened by two hours. From this result, it became clear that fermentation time could be shortened by using the present technique.

### <Experimental Example 2>

In Experimental Example 2, the effects of using hemicellulase and cellulase on the fermentation time during fermentation of plant-base raw materials were investigated. Soy milk was used as the plant-base raw material.

### (1) Experimental method

Each 10 mL of soy milk ("Organic Soy Milk with No Adjustment of Ingredients" manufactured by Marusan-Ai Co., Ltd.) was dispensed into 15 mL tubes, and hemicellulase derived from Aspergillus niger ("Hemicellulase 'Amano' 90" manufactured by Amano Enzyme Inc.) was added to be 0.1 (w/v)% (90 U/mL) or cellulase derived from Aspergillus niger ("Cellulase A 'Amano' 3" manufactured by Amano Enzyme Inc.) was added to be 0.1 (w/v)%, and mixed. To the soy milk after enzyme addition, 0.2 mL of lactic acid bacteria starter containing Lactococcus delbrueckii subsp. delbureckii was added, fermentation was performed overnight at 37°C, and the pH was confirmed using a pH sensor after 3, 6, and 10 hours. Note that the example in which no enzyme was added was designated as an enzyme-free group.

### (2) Results

The results are shown in Table 2.

**[Table 2]**

| Enzyme | 3 hrs | 6 hrs | 10 hrs |
|---|---|---|---|
| Enzyme-free | 6.59 | 6.33 | 5.61 |
| Cellulase | 6.33 | 6.1 | 4.87 |
| Hemicellulase | 6.21 | 5.41 | 3.97 |

### (3) Consideration

As shown in Table 2, when the hemicellulase treatment was performed, the rate of pH reduction was much faster than in the enzyme-free group. In addition, in the cellulase-added group, an improvement in the rate of pH reduction was observed compared to the enzyme-free group, but the effect of improving the pH-decrease rate was more remarkable in the hemicellulase-added group.

### <Experimental Example 3>

In Experimental Example 3, the effects on stringiness of using hemicellulase and using cellulase during fermentation of plant-base raw materials were investigated. Soy milk was used as the plant-base raw material.

### (1) Experimental method

Into a sterilized 300 mL beaker, 280 mL of soy milk ("Organic Soy Milk with No Adjustment of Ingredients" manufactured by Marusan-Ai Co., Ltd.) was dispensed, and after cooling with water, 5.6 mL of lactic acid bacteria starter containing Lactococcus delbrueckii subsp. delbureckii, and hemicellulase derived from Aspergillus niger ("Hemicellulase 'Amano' 90" manufactured by Amano Enzyme Inc.) was added to be 0.1 (w/v)% (90 U/mL) or cellulase derived from Aspergillus niger ("Sumizyme AC" manufactured by Shinnihon Chemicals Corp.) was added to be 0.1 (w/v), in a clean bench, and fermentation was carried out at 37°C overnight. After fermentation, the outflow time was measured using a Zahn cup for the stringiness of the soy milk yogurt. Note that the example in which no enzyme was added was designated as an enzyme-free group.

### (2) Results

The results are shown in Table 3.

**[Table 3]**

| Enzyme | Outflow time |
|---|---|
| Enzyme-free | 6 sec |
| Cellulase | 30 sec |
| Hemicellulase | 67 sec |

### (3) Consideration

As shown in Table 3, the outflow time was 6 seconds in the enzyme-free group, but the outflow time was 67 seconds in the hemicellulase-added group, showing a dramatic improvement in stringiness. Furthermore, in the cellulase-added group, an improvement in stringiness was observed compared to the enzyme-free group, but the effect of improving stringiness was more remarkable in the hemicellulase-added group. From this result, it has become clear that the use of the present technique improves the stringiness.

### <Experimental Example 4>

In Experimental Example 4, the effect of different amounts of hemicellulase added on the effect of improving stringiness was investigated. Soy milk was used as the plant-base raw material.

### (1) Experimental method

Into a sterilized 300 mL beaker, 280 mL of soy milk ("Organic Soy Milk with No Adjustment of Ingredients" manufactured by Marusan-Ai Co., Ltd.) was dispensed, and after cooling with water, 5.6 mL of lactic acid bacteria starter containing Lactococcus delbrueckii subsp. delbureckii was added, and hemicellulase derived from Aspergillus niger ("Hemicellulase 'Amano' 90" manufactured by Amano Enzyme Inc.) was added in an amount shown in Table 4 below, in a clean bench, and fermentation was carried out overnight at 37°C. After fermentation, the outflow time was measured using a Zahn cup for the stringiness of the soy milk yogurt.

### (2) Results

The results are shown in Table 4.

**[Table 4]**

| Amount of hemicellulase | | Outflow time |
|---|---|---|
| (w/v)% | (U/mL) | |
| 0.001 | 0.9 | 23 sec |
| 0.01 | 9 | 80 sec |
| 0.1 | 90 | 64 sec |
| 1 | 900 | 58 sec |

### (3) Consideration

As shown in Table 4, compared to when the amount of hemicellulase added was 0.9 U/mL, when the amount of hemicellulase added was 9 U/mL or more, the outflow time was significantly extended. From this result, it was found that in order to reliably improve the stringiness, it is preferable to add 8 U/mL or more of hemicellulase.

## Claims

1. A modifier for fermented plant-base drinks or foods, the modifier comprising hemicellulase.

2. The modifier for fermented plant-base drinks or foods according to claim 1, which has an effect of promoting fermentation.

3. The modifier for fermented plant-base drinks or foods according to claim 1, which has an effect of improving stringiness.

4. The modifier for fermented plant-base drinks or foods according to any one of claims 1 to 3, wherein the hemicellulase is a hemicellulase derived from a microorganism.

5. The modifier for fermented plant-base drinks or foods according to any one of claims 1 to 3, wherein the fermented plant-base drink or food is a fermented plant-base milk or a plant-base lactic acid bacteria drink.

6. A method for producing a fermented plant-base drink or food, the method comprising:
an enzymatic action step for allowing hemicellulase to act on a portion or all of a plant-base raw material; and
a fermentation step for performing fermentation.

7. The method for producing a fermented plant-base drink or food according to claim 6, wherein the enzymatic action step is performed before and/or simultaneously with the fermentation step.

8. The method for producing a fermented plant-base drink or food according to claim 6 or 7, wherein lactic acid fermentation is performed in the fermentation step.

9. A method for promoting fermentation of a fermented plant-base drink or food, the method comprising:
an enzymatic action step for allowing hemicellulase to act on a portion or all of a plant-base raw material; and
a fermentation step for performing fermentation.

10. A method for improving the stringiness of a fermented plant-base drink or food, the method comprising:
an enzymatic action step for allowing hemicellulase to act on a portion or all of a plant-base raw material; and
a fermentation step for performing fermentation.
